Europäisches Patentamt

European Patent Office

Office européen des brevets

(11) Numéro de publication: **0 237 401**
**A1**

## (12) DEMANDE DE BREVET EUROPEEN

(21) Numéro de dépôt: 87400422.9

(22) Date de dépôt: 25.02.87

(51) Int. Cl.⁴: **C 07 K 15/04**
C 07 K 3/02, A 61 K 39/02,
C 12 P 21/00, G 01 N 33/531,
G 01 N 33/574

(30) Priorité: 26.02.86 FR 8602690

(43) Date de publication de la demande:
16.09.87 Bulletin 87/38

(84) Etats contractants désignés:
AT BE CH DE ES FR GB GR IT LI LU NL SE

(71) Demandeur: **CENTRE NATIONAL DE LA RECHERCHE
SCIENTIFIQUE (CNRS)**
**15, Quai Anatole France**
**F-75007 Paris (FR)**

(72) Inventeur: **Kohiyama, Masamichi**
**7, rue P. Sémard**
**F-75009 Paris (FR)**

**Ben Mahrez, Kamel**
**Maison de Tunisie Ch. 229 45 Boulevard Jourdan**
**F-75014 Paris (FR)**

**Thierry, Dominique**
**10 rue de la Mare**
**F-75020 Paris (FR)**

(74) Mandataire: **Peaucelle, Chantal et al**
**S.C. Ernest Gutmann - Yves Plasseraud 67, boulevard
Haussmann**
**F-75008 Paris (FR)**

(54) **Protéines homologues de celles codées par des oncogènes humains, procédé d'obtention de ces protéines et leurs
applications immunologiques, physiopathologiques et thérapeutiques.**

(57) Les protéines de l'invention sont caractérisées en ce
qu'elles sont reconnues spécifiquement par des anticorps
anti-c-myc humain.

EP 0 237 401 A1

**Description**

PROTEINES HOMOLOGUES DE CELLES CODEES PAR DES ONCOGENES HUMAINS, PROCEDE
D'OBTENTION DE CES PROTEINES ET LEURS APPLICATIONS IMMUNOLOGIQUES
PHYSIOPATHOLOGIQUES ET THERAPEUTIQUES.

L'invention concerne des protéines homologues de celles codées par des oncogènes humains, un procédé d'obtention de ces protéines et leurs applications immunologiques et thérapeutiques.

Elle vise plus spécialement l'élaboration des produits pour des tests immunologiques, spécifiques vis-à-vis de protéines exprimées par l'oncogène c-myc humain ou vis-à-vis d'anticorps reconnaissant ces protéines.

L'expression de l'oncogène c-myc est liée à plusieurs types de tumeurs humaines. Un antisérum polyclonal contre myc, fabriqué par injection d'un peptide synthétique chez les lapins, est actuellement commercialisé. Cependant, le coût de cet antisérum est élevé. Une élaboration d'antisérum simplifiée contre la protéine c-myc humaine constituerait un progrès déterminant pour le dépistage ou le diagnostic d'un cancer.

Des travaux des inventeurs sur les archaebactéries, en particulier sur Halobacterium halobium chez qui les inventeurs ont trouvé un gène dont la séquence nucléotidique (1 Kbp) possède environ 50% d'homologie avec le gène v-myc aviaire, les ont conduits à isoler une protéine immunologiquement apparentée à celle de c-myc, apportant ainsi une solution au problème évoqué plus haut.

L'invention a donc pour but de fournir des protéines similaires à la protéine c-myc humaine.

Elle vise également un procédé d'obtention de cette protéine à partir de cultures d'archaebactéries, plus spécialement d'Halobacterium halobium.

L'invention vise en outre les applications immunologiques, physiopathologiques et thérapeutiques, en particulier l'utilisation des anticorps spécifiques formés contre les protéines ou l'utilisation de ces protéines comme réactifs permettant d'identifier respectivement les protéines exprimées par c-myc humain ou les anticorps formés chez un sujet.

Les protéines de l'invention sont caractérisées en ce qu'elles sont reconnues spécifiquement par des anticorps anti-c-myc humain.

Des protéines de ce type sont d'origine archaebactérienne.

Grâce aux travaux des inventeurs, il apparaît que la séquence d'ADN des archaebactéries codant pour ces protéines possède une similarité suffisante avec celle de c-myc humain pour qu'on dispose, dans les protéines respectivement exprimées, d'épitopes homologues.

Une protéine archaebactérienne préférée est exprimée par Halobacterium halobium.

Il s'agit plus spécialement d'une protéine ayant un poids moléculaire d'environ 84 KD.

L'invention vise également un procédé d'obtention de ces protéines.

Selon ce procédé, on traite la matière insoluble d'un extrait d'archaebactéries obtenu après broyage afin de séparer les protéines de l'ADN auquel elles sont fixées, puis on élimine au moins la majeure partie des protéines acides par contact avec un échangeur d'ions, après la sonication de ce matériel.

Selon un mode préféré de réalisation du procédé, on soumet une culture d'archaebactéries à une opération de broyage en milieu tampon et on récupère le produit insoluble.

Il est avantageux d'effectuer au moins une étape de centrifugation afin d'assurer une séparation satisfaisante de la matière insoluble.

La matière insoluble récupérée renferme les protéines recherchées fixées à l'ADN.

Pour détacher les protéines du complexe à l'ADN, on a recours à un traitement, tel qu'une opération de sonication, qui permet de fragmenter les chaînes d'ADN auxquelles sont fixées les protéines. Pour éliminer les chaînes d'ADN de ces fragments, on a recours par exemple à l'action d'une ADN ase.

La sonication est avantageusement réalisée en un milieu tampon. Les fragments obtenus sont solubles dans le milieu tampon et sont avantageusement séparés des matières insolubles présentes par centrifugation.

Le surnageant qui renferme donc ces fragments solubles est traité alors à l'aide d'ADN ase pour éliminer les fragments d'ADN des fragments de protéines.

La préparation de protéines obtenue est soumise à au moins une étape de purification afin d'éliminer pratiquement tout l'ADN du milieu.

A cet effet, on a recours avantageusement à une chromatographie sur une résine échangeuse d'ions par exemple du type DEAE cellulose.

La fraction non adsorbée qui renferme les protéines recherchées est recueillie. Cette purification peut être complétée en utilisant un échangeur de cations tel que de l'hydroxyapatite. Une purification supplémenttaire est réalisée, le cas échéant, par exemple par mise en contact avec un colorant tel que celui commercialisé par Pharmacia sous la marque Bleu Sépharose.

L'invention vise également les applications immunologiques, physiopathologiques et thérapeutiques des protéines définies ci-dessus.

La présence chez ces protéines d'épitopes similaires àceux des protéines codées par c-myc est exploitée pour l'élaboration de produits immunologiques.

L'utilisation de ces protéines pour former des antisera anti c-myc constitue une nouvelle voie de préparation de tels produits. Cette voie consiste à injecter à des animaux, selon les techniques classiques, par exemple aux lapins, la protéine de l'invention analogue à celle exprimée par c-myc.

L'invention vise donc les antisera renfermant les anticorps spécifiques contre la protéine de c-myc. Elle vise également les anticorps polyclonaux et monoclonaux capables de reconnaître spécifiquement la protéine c-myc et par là de permettre la mise en évidence de l'expression de cette protéine chez un patient par simple test immunologique.

Les protéines de l'invention per se constituent de précieux réactifs immunologiques susceptibles de mettre en évidence un auto-immunité, à savoir la présence d'anticorps anti-c-myc humain dans le sérum d'un sujet, ce qui peut être utilisé, par exemple, pour étudier la variation du taux d'anticorps anti-c-myc d'un patient en cours de chimiothérapie ou le dépistage de la présence tumorale.

L'invention fournit, d'autre part, un test immunologique permettant le dépistage préliminaire d'un cancer. Ce test consiste à utiliser directement la protéine archaebacterienne comme antigène contre l'anticorps anti-c-myc humain qui est présent dans des serums de certains malades cancereux.

L'avantage de ce test consite à éviter la purification de l'antigène c-myc. En effet, on peut effectuer l'immunoblotting avec un échantillon non purifié de la protéine 84 KD de Halobacterium halobium. Le même procédé est difficilement réalisable avec un extrait de lignée cellulaire humaine exprimant c-myc par exemple HL60 à cause de réactions immunologiques multiples.

Pour la mise en oeuvre de ces applications, les produits de l'invention se présentent avantageusement sous forme de kits renfermant les éléments nécessaires à la réalisation du test. En particulier, l'invention vise des kits renfermant une protéine d'environ 84 KD telle que définie ci-dessus fixée sur un support ou l'anticorps polyclonal ou monoclonal correspondant, un colorant et un réactif tel que l'anti-Ig correspondant conjugué à la peroxydase, pour la mise en évidence de la réaction immunologique.

Selon un autre aspect de grand intérêt, les protéines de l'invention sont utilisables aux fins d'immunisation d'un patient.

Les vaccins élaborés à partir de ces protéines entrent donc également dans le cadre de l'invention.

D'autres caractéristiques et avantages de l'invention apparaîtront dans les exemples qui suivent et en se référant aux figures 1 à 4,

    - les figures 1 et 2 représentent des photos d'un immunoblotting montrant la présence de la protéine 84 KD

    - la figure 3 représentant la photo d'un immunoblotting avec l'Ig-anti 84 KD et la protéine c-myc humaine, et

    - la figure 4 représente la photo d'un test immunologique.

EXEMPLE 1 -

Procédé d'obtention et purification d'une protéine de Halobacterium halobium analogue à celle codée par l'oncogène c-myc humain.

On utilise un extrait d'Halobacterium halobium contenant $10^9$ cellules par ml de culture, ces cellules étant en phase exponentielle de croissance.

On désintègre les cellules à l'aide d'un homogénéiseur du type POTTER dans un tampon constitué par 25 mM d'Hepes pH 7,5, 0,5mM d'EDTA, 1mM de dithiothréitol, 3M de KCl et 1mM de PMSF.

On soumet le broyat à une centrifugation durant 1 h à 45.000 t/mn. Le culot est repris et centrifugé dans les mêmes conditions.

On met en suspension le culot récupéré dans 10mM de $Na_2HPO_4$ pH 6,8, 10% de glycérol, $5 \times 10^{-4}$M d'EDTA et 1mM de PMSF.

La suspension est soumise à une étape de sonication, à 6 reprises, durant 15 secondes, puis on centrifuge 10mn à 18000 t/mn.

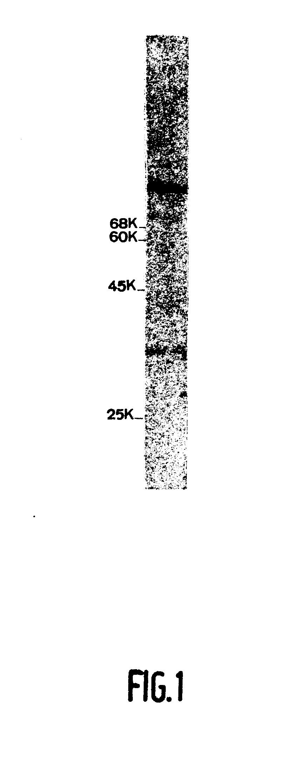

L'immunoblotting de ce surnageant montre la présence d'une protéine de 84 KD de Halobacterium halobium qui réagit avec l'anticorps anti-c-myc humain. -(fig. 1).

Le surnageant est traité avec de l'ADNase (10 µg/ml) en présence de 0,01M de $MgCl_2$, à 37°C, durant 1 heure et demie.

On purifie, la fixation soluble obtenue, sur une colonne de DEAE cellulose équilibrée avec un tampon renfermant 0,02M de $Na_2HPO_4$, pH 7,3, 0,1M de KCl et 10% de glycérol.

On recueille la fraction non adsorbée et on la soumet à une autre étape de purification par passage sur une colonne d'hydroxylapatite, équilibrée avec un tampon 0,01M de $Na_2HPO_4$, pH 6,8, 0,1M de KCl, et 10% de glycérol.

On élue la colonne avec un gradient de $Na_2$ $Na_2HPO_4$, de 0,05M à 0,5M.

On soumet l'éluat obtenu à une étape de dialyse contre un tampon de Bleu Sépharose. Pour une purification supplémentaire, on fait passer le dialysat à travers une colonne de Bleu Sépharose équilibrée avec un tampon formé de 0,02M de $Na_2HPO_4$ pH 6,8, 0,1M de KCl et 10% de glycérol.

On élue avec un gradient de NaCl dans ωε tampon de 0,1M à 5M.

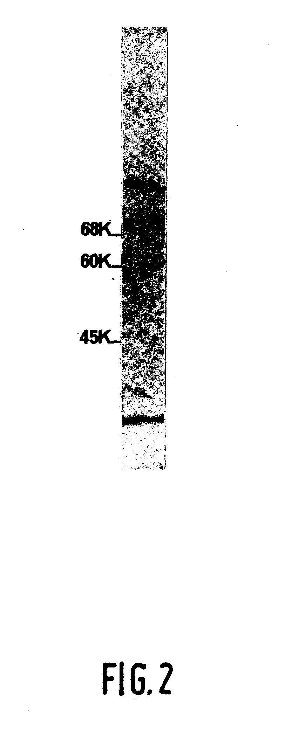

On récupère l'éluat qui contient la protéine apparentée à celle codée par c-myc. (voir fig. 2)

- Purification de la protéine de 84 KD et obtention d'un antisérum.

On prépare les anticorps en injectant la protéine de 84 KD purifiée selon les techniques ci-dessus. On injecte la protéine purifiée à des lapins, à raison de 100 ug de protéine par animal dans l'adjuvant complet de Freund. L'injection est répétée un mois après et l'antiserum récupéré selon les techniques classiques. 15 jours après l'injection.

Cet Ig-anti-84 KD réagit avec la protéine c-myc humaine.

L'immunoblotting montre un peptide de 55-60 KD présent dans l'extrait de HL 60 et absent dans l'extrait de la même lignée cellulaire traitée au DMSO. Ce même peptide est présent dans l'extrait de OD262 et absent chez MCF-7.

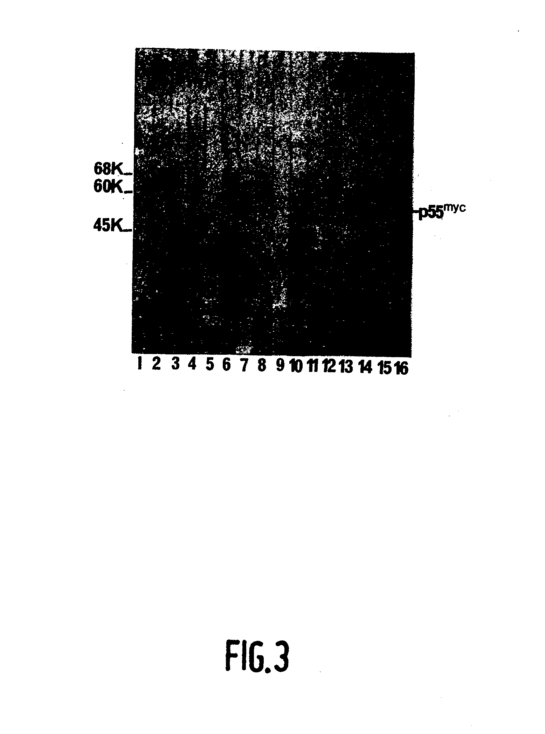

Comme le montre la photo de la figure 3, l'expérience d'immunoblotting conduit à une réaction entre l'Ig-anti 84 KD et la protéine c-myc humaine dans les lignées HL 60 et OD 262.

Sur cette figure, les bandes numérotées 1 à 15 représentent:

1 - HL60 + DM 50 + Ig c-myc Oncor
2 - HL60 + DM 50 + Ig 70 KD
3 - HL60 + DM 50 + Ig 84 KD
5 - HL60 + Ig c-myc Onc
6 - HL60 + Ig 70 KD
7 - HL60 + Ig 84 KD
9 - CD 262 + Ig-c-myc Onc
10 - CD 262 + Ig-70 KD
11 - CD 262 + Ig-84 KD
13 - MCF 7 + Ig-c-myc Onc
14 - MCF 7 + Ig-70 KD
15 - MCF 7 + Ig-84 KD

L'expérience effectuée sur les extraits des noyaux des cellules HL60 traitées ou non, démontre clairement que l'anticorps anti-84 KD reconnaît la protéine c-myc (55-60 KD).

Cette protéine présente dans HL60 non traitée est également reconnue par l'anticorps anti-c-myc (Oncor).

EXEMPLE 2 -

Détection de l'Ig anti-c-myc humain.

Une bande positive du test peroxydase est localisée à la hauteur de 84 KD mettant en évidence la présence dans le serum du sujet de l'anticorps anti-c-myc humain.

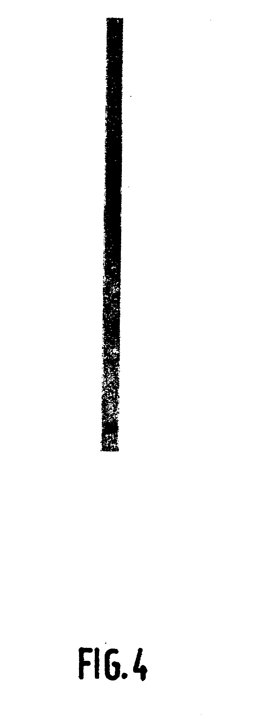

Sur la figure 4, on montre la photo d'un test immunologique effectué à l'aide du sérum d'un sujet normal.

Reconnaissance de la protéine de 84 KD par des serums humains

On transfère une préparation de protéine de 84KD sur filtre de nitrocellulose Millipore®. On détecte la présence d'anticorps contre cette protéine dans le serum de patients atteints du cancer et de sujets normaux.

Sur les 212 serums testés, 21 reconnaissent la protéine de 84KD. Pour confirmer la validité du test, plusieurs sérums positifs et négatifs ont été testés avec les préparations d'extraits de HL-60.

Les résultats obtenus montrent que les serums positifs contiennent des anticorps dirigés contre un épitope que partagent la protéine de 84 KD de Halobacterium halobium et la protéine c-myc humaine de 60 KD. Aucune réponse positive n'est exprimée avec 41 serums normaux étudiés. Les serums positifs proviennent très fréquemment de patients avec des cancers colorectaux.

Les résultats ci-après obtenus sont rapportés sur le tableau ci-après:

| Type de cancer | nombre de réponses positives | nombre de réponses négatives |
|---|---|---|
| Sein (adenocarcinome) | 12 | 113 |
| Mélanome | O | 5 |
| Colon | 4 | 2 |
| ORL | O | 3 |
| Ovaires | 1 | 8 |
| Hodgkin | O | 8 |
| Foie | O | 1 |
| Ostérosarcome | 1 | 1 |
| Utérus | O | 2 |
| Teratocarcinome | O | 1 |
| Neuroblastome | O | 2 |
| Cancer primaire inconnu | 3 | O |

L'utilisation selon l'invention de la protéine de 84 KD définie ci-dessus, est particulièrement efficace pour la détection de cancers du colon.

## Revendications

1/ Protéines homologues de celles codées par des oncogènes humains caractérisées en ce qu'elles sont reconnues spécifiquement par des anticorps anti-c-myc humain.

2/ Protéines selon la revendication 1, caractérisées en ce qu'elles sont d'origine archaebactérienne.

3/ Protéine exprimée par Halobacterium halobium ayant un poids moléculaire d'environ 84KD possédant au moins un épitope reconnu spécifiquement par des anticorps anti-c-myc humain.

4/ Procédé d'obtention de protéines selon l'une quelconque des revendications 1 à 3, caractérisé en ce qu'on traite la matière insoluble d'un extrait d'archaebactéries obtenu après broyage afin de séparer les protéines de l'ADN auquel elles sont fixées, puis on élimine au moins la majeure partie, pratiquement la totalité, des protéines acides par contact avec un échangeur d'ions.

5/ Procédé selon la revendication 4, caractérisé en ce que la séparation des protéines de la matière insoluble comprend une étape de sonication.

6/ Procédé selon la revendication 4 ou 5, caractérisé en ce qu'il comprend les étapes suivantes:

- on soumet une culture d'archaebactéries à une opération de broyage en milieu tampon,

- on soumet le produit insoluble obtenu à au moins une étape de centrifugation,

- on soumet le culot de centrifugation à une étape de sonication qui conduit à la solubilisation de fragments renfermant les protéines complexées à l'ADN,

- on élimine les matières insolubles par centrifugation et on récupère le surnageant,

- on traite le surnageant avec de l'ADNase, puis

- on effectue au moins une étape de purification de la préparation de protéine par exemple par chromatographie sur une résine échangeuse d'ions puis sur une résine échangeuse de cations.

7/ Produits immunologiques caractérisés en ce qu'ils sont élaborés à partir des protéines selon l'une quelconque des revendications 1 à 3.

8/ Antisérum anti c-myc humain caractérisé en ce qu'il est obtenu à partir de sera d'animaux auxquels on a injecté des protéines selon l'une quelconque des revendications 1 à 3.

9/ Anticorps monoclonaux ou polyclonaux formés contre les protéines selon l'une quelconques des revendications 1 à 3.

10/ Application d'une protéine selon l'une quelconque des revendications 1 à 3 à l'élaboration de vaccins pour immunisation de patients contre c-myc humain.

11/ Kits pour tests immunologiques caractérisés en ce qu'ils renferment une protéine selon l'une quelconque des revendication 1 à 3, en particulier la protéine d'environ 84 KD selon la revendication 3, ou un anticorps polyclonal ou monoclonal correspondant, et les éléments nécessaires pour la mise en évidence d'une réaction immunologique tels qu'un colorant et une peroxydase anti-Ig humaine complexée.

0237401

FIG.1

0237401

68K_
60K_
45K_

FIG.2

68K—
60K—

45K—

p55^myc

1 2 3 4 5 6 7 8 9 10 11 12 13 14 15 16

# FIG.3

0237401

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 108 564 (THE REGENTS OF THE UNIVERSITY OF CALIFORNIA) * Page 3, ligne 15 - page 4, ligne 13; page 4, lignes 20-29; page 8, lignes 8-21,23 - page 9, ligne 6; page 11, lignes 4-18; page 13, lignes 1-12; revendications 1-5,14,17,18,19,21,22,29 * | 1,8,9, 11 | C 07 K  15/04<br>C 07 K   3/02<br>A 61 K  39/02<br>C 12 P  21/00<br>G 01 N  33/531<br>G 01 N  33/574 |
|  | --- |  |  |
| A | CHEMICAL ABSTRACTS, vol. 103, no. 3, 22 juillet 1985, page 134, résumé no. 17577m, Columbus, Ohio, US; B. PERBAL et al.: "Sequences homologous to the v-myb oncogene in the genome of archaebacteria" & C. R. ACAD. SCI., SER, 3 1985, 300(5), 177-80 * Résumé * | 1-3 |  |
|  |  |  | DOMAINES TECHNIQUES RECHERCHES (Int. Cl.4) |
|  | --- |  |  |
| X,P | EP-A-0 206 942 (CENTRE NATIONAL DE LA RECHERCHE SCIENTIFIQUE) * Page 1, lignes 7-10; page 2, lignes 1-9,17-24,28 - page 3, ligne 1; page 3, lignes 5-11,30 - page 6, ligne 15; revendications 4,6,11,12 * | 1,2,4, 7-9 | A 61 K<br>G 01 N<br>C 12 P<br>C 12 N |
|  | ----- |  |  |

Le présent rapport de recherche a été établi pour toutes les revendications

| Lieu de la recherche | Date d'achèvement de la recherche | Examinateur |
|---|---|---|
| LA HAYE | 10-06-1987 | CHARLES D.J.P.I.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82